# EUROPEAN PATENT APPLICATION

(11) **EP 4 335 473 A1**
(43) Date of publication of application: **13.03.2024**
(21) Application number: 22194232.9
(22) Date of filing: 06.09.2022
(51) Int. Cl.: A61M 5/20, A61M 5/315, A61M 5/32, A61M 37/00

(54) **AN AUTOMATED INJECTION SYSTEM**

(71) Applicant: Latch Medical Limited, 4 Dublin (IE)
(72) Inventor: Bertollo, Nicky, Dublin, 24 (IE)
(74) Representative: FRKelly

(57) **Abstract**

The present invention provides an automated injection system for transdermal or intradermal delivery of a medicament or the like which provides a robust yet greatly simplified means of operation, the system including an elongate housing forming a handle at one end of which is located an array of microneedles, the injection system further comprising an internal reservoir of the medicament to be injected through the microneedles, and an actuator assembly contained within the housing which is arranged to allow the injection system to be operated by way of a simple one handed grip and press action by the user to both insert the microneedles into the skin and automatically inject the medicament.

## Description

### Field of the Invention

The present invention relates to an automated injection system, and in particular an injection system for transdermal or intradermal delivery of a medicament or the like which provides a robust yet greatly simplified means of operation and thus to allow for reliable self-injection.

### Background of the invention

Chronic diseases and conditions are becoming more prevalent, due in part to changing social behaviours and ageing populations. Therapeutic treatment using biologics (including antibodies, therapeutic proteins, peptides and cell-based therapies) offers distinct advantages in efficacy and selectivity, and their application in the therapeutic areas of cancer and cancer related diseases, rare diseases, neurological disorders and autoimmune diseases is increasing. However, the specific physiochemical properties of biologics (including high viscosity) mean that delivering them as active agents into the body can be challenging, necessitating either intravenous (IV) or subcutaneous administration. The advent of patient-centric "autoinjector" designs and devices allow users across chronic disease states to routinely self-inject drugs subcutaneously at home, which is advantageous as it eliminates the need to present at a hospital or an infusion centre.

Existing subcutaneous autoinjectors nevertheless have several limitations. In these prior art designs, medicament is typically administered via a single needle inserted perpendicularly through the skin. A common approach to needle insertion involves utilising potential energy stored in a spring, for example, to propel the needle with considerable speed and force through the skin. In addition to causing potential pain and discomfort for the patient this actioncan sometimes result in needle breakage. Depending on such factors as volume and viscosity of the medicament a "hold time" from seconds up to minutes can then be required for the automated injection to be administered (and which usually requires a second stored potential energy source). For older patients and those with dexterity issues and/or tremors maintaining a constant, steady hand during this injection phase may present a challenge. Highly viscous biologics are known to be associated with a long injection and "hold times". One strategy to decrease injection time and thus "hold time" by reformulating medicaments to be less viscous whilst retaining payload however necessitates higher injection volumes, placing further demands on device engineering as well impacting the patient experience.

Skin as a delivery site (intradermal delivery) for both biologics and vaccines offers unparalleled efficiency (i.e. dose-sparing) and efficacy (i.e. dose response).

However this form of delivery, using the so-called Mantoux Technique, can be difficult or time consuming to perform for various reasons such as the experience of the administrator and the accuracy required to correctly perform the procedure, the location and/or condition of the delivery site, finite thickness of the skin and the required depth of delivery, etc. As a result this form of injection is generally not suited to self-injection. This is particularly the case for individuals with reduced dexterity, poor eyesight, or other physical impairment which may increase the difficulty of performing the convention task of intradermal injection. Delivery of highly viscous medicaments also presents a considerable challenge using a single needle.

There is therefore a need for an intradermal injection system which can be used for self-injection and which is simple to use, reliable, and repeatable. There is a further need for an injection system that can deliver vaccines and viscous biologics in a rapid fashion with reduced injection pressure and which can be easily handled by all patient types.

It is therefore an object of the present invention to address the above mentioned problem.

### Summary of the invention

According to the present invention there is provided an automated injection system comprising an elongate housing defining a handle; an actuator assembly disposed within the housing and displaceable longitudinally relative to the housing; an injection head mounted to the actuator assembly and projecting from a lower open end of the housing, the injection head comprising a pair of jaws displaceable relative to one another in a direction transverse to a longitudinal axis of the housing and in response to the relative longitudinal displacement between the housing and the actuator assembly, each jaw having one or more hollow microneedles projecting therefrom; a reservoir of liquid contained within the housing in fluid communication with one or more of the hollow microneedles; and a dispenser operable to pressurise the fluid in the reservoir in response to the relative longitudinal displacement between the housing and the actuator assembly in order to dispense the liquid from the one or more hollow microneedles.

Preferably, the reservoir is defined by a syringe comprising a cylinder closed at one end by a piston displaceable into the cylinder by the dispenser in order to pressurise the fluid.

Preferably, the dispenser comprises a spring biased plunger in operative association with the piston.

Preferably, the plunger comprises an abutment and the actuator assembly comprises a latch member against which the abutment is releasably retained.

Preferably, the actuator assembly is operable to effect displacement of the latch member out of engagement with the abutment in sequence prior to or following the completion of relative transverse displacement of the pair of jaws.

Preferably, the actuator assembly comprises a biasing element arranged to urge the latch member out of engagement with the abutment following the relative longitudinal displacement between the housing and the actuator assembly.

Preferably, the biasing element comprises a guide member fixed to the housing and displaceable against the latch member in response to the relative longitudinal displacement between the housing and the actuator assembly such as to urge the latch member out of engagement with the abutment.

Preferably, one of the jaws and the housing comprise a cam and the other of the jaws and the housing comprise a corresponding follower, the cam having a profile arranged to effect the relative transverse displacement of the pair of jaws in response to the relative longitudinal displacement between the housing and the actuator assembly.

Preferably, each jaw comprises a cam and the housing comprising a corresponding pair of followers.

Preferably, the or each cam is profiled to permit a two stage displacement of the jaws, a first stage configured to effect the relative transverse displacement of the jaws in response to the relative longitudinal displacement between the housing and the actuator assembly, and a second stage permitting the relative movement between the jaws without relative longitudinal displacement between the housing and the actuator assembly.

Preferably, the or each cam is profiled to define a third phase between the first and second phase which permits the relative longitudinal displacement between the housing and the actuator assembly in the absence of relative transverse displacement of the jaws.

Preferably, the injection head comprises at least one release member manually operable to reverse the relative transverse displacement between the pair of jaws.

Preferably, the housing comprises an aperture in a sidewall through which the release member is accessible following the relative longitudinal displacement between the housing and the actuator assembly.

Preferably, the injection system comprises a locking element operable to prevent reversal of the relative longitudinal displacement between the housing and the actuator assembly.

Preferably, the actuator assembly comprises an elongate frame about which the housing is supported and within which the reservoir is contained, the injection head being mounted at an end of the frame.

Preferably, the hollow microneedles on each jaw are inclined towards the hollow microneedles on the other jaw.

Preferably, the injection system comprises a cap releasably securable about the lower end of the housing to enclose the injection head.

Preferably, the automated injection system comprising a spring arranged to urge the housing longitudinally of the actuator assembly.

Preferably, the spring comprises a leaf spring disposed between the actuator assembly and a closed end of the housing.

### Brief Description of the Drawings

The present invention will now be described with reference to the accompanying drawings, in which:
Figure 1a illustrates a front elevation of an embodiment of an automated injection system according to the present invention in an undeployed state;
Figure 1b illustrates the automated injection system of Figure 1 in a deployed state;
Figure 1c illustrates the automated injection system of Figures 1 and 2 in a release state;
Figure 2a illustrates a sectioned view of the injection system as shown in Figure 1a;
Figure 2b illustrates a sectioned view of the injection system as shown in Figure 1b;
Figure 2c illustrates the injection system as shown in Figure 1b following actuation of a syringe;
Figure 2d illustrates a sectioned view the injection system as shown in Figure 1c;
Figures 3a, 3b and 3c illustrate enlarged views of an injection head of the automated injection system as shown in Figures 1a, 1b and 1c;
Figures 4a, 4b and 4c illustrate an actuation assembly forming part of the automated injection system as shown in Figures 1 to 3 in partially exploded views and an assembled view;
Figure 5 illustrates a perspective view of the automated injection system of Figures 1 to 4 in the undeployed state;
Figure 6 illustrates an exploded perspective view of the automated injection system of Figures 1 to 5;
Figures 7a, 7b and 7c illustrate various exploded perspective views of the injection head and syringe coupling forming part of the automated injection system of Figures 1 to 6;
Figures 8a, 8b and 8c illustrate various front elevations of the injection head and syringe coupling as seen in Figures 7a, 7b and 7c;
Figure 9 illustrates a perspective view of an alternative embodiment of an automated injection system according to the present invention in an undeployed state;
Figure 10 illustrates a perspective view of the alternative embodiment of Figure 9 in a deployed state;
Figure 11a illustrates a sectioned view of the automated injection system as shown in Figure 9;
Figure 11b illustrates a sectioned view of the automated injection system as shown in Figure 10;
Figure 11c illustrates the automated injection system of Figures 9 and 10 in a release state;
Figure 12a illustrates a front elevation of a further alternative embodiment of an automated injection system according to the present invention and in an undeployed state;
Figure 12b illustrates a sectioned view of the automated injection system as shown in Figure 12a;
Figure 12c illustrates the automated injection system of Figure 12b in a deployed state;
Figure 13a illustrates a sectioned perspective view of the automated injection system as shown in Figure 12a and 12b;
Figure 13b illustrates one half of a housing of the automated injection system as seen in Figure 13a and in an undeployed state;
Figure 13c illustrates the housing of Figure 13b in a deployed state;
Figure 14 illustrates a perspective view of a two part automated injection system according to a still further alternative embodiment of the present invention and in a partially separated state;
Figure 15 illustrates a front elevation of the injection system of Figure 14; and
Figure 16 illustrates an assembled view of the injection system shown in Figures 14 and 15.

### Detailed Description of the Drawings

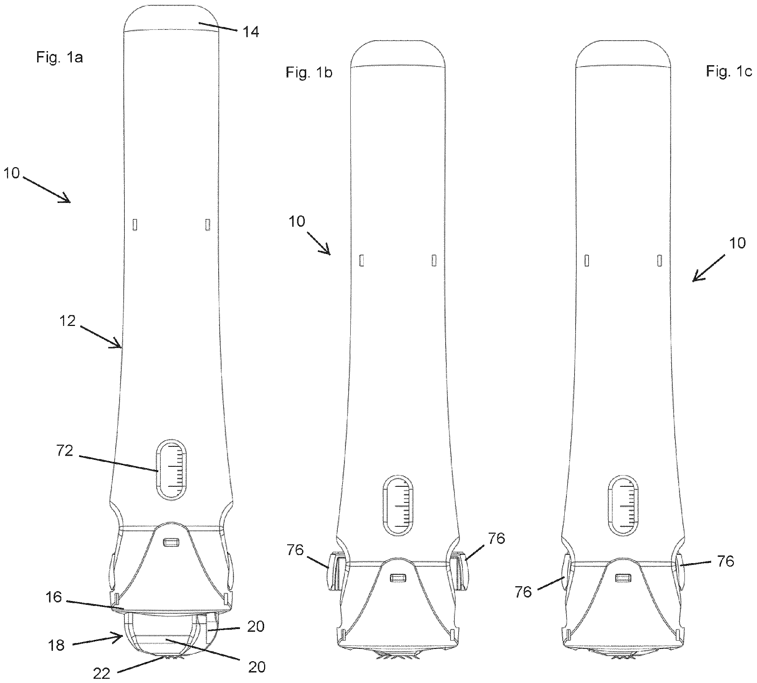

Referring now to Figures 1 to 8 of the accompanying drawings there is illustrated an automated injection system, generally indicated as 10, which is operable to provide a simple yet consistent means of injecting a fluid medicament (not shown) or the like into tissue, in particular intradermal delivery, and most preferably allowing such injections to be performed using a single hand and therefore to permit self-injection.

The injection system 10 comprises an elongate and substantially cylindrical housing 12 which in use defines a pistol grip type handle by which the injection system 10 is held and operated, the length and diameter of the housing 12 thus being dimensioned accordingly for a comfortable fit within a user's hand. The housing 12 may be formed from any suitable material or combination of materials, and for example may be formed by injection moulding of plastic. The housing 12 comprises a closed upper end 14 and an open lower end 16 from which extends an injection head 18. The injection head 18 comprises a pair of opposed jaws 20 from a lower face of each of which project a number of hollow microneedle 22 which are inclined in a direction towards the opposed jaw 20. The injection system 10 is operable, as described in detail hereinafter, to automatically insert the array of microneedles 22 into the skin or other tissue, and to then inject a liquid medicament when the housing 12 is gripped by the user and the injection head 18 pressed against the skin, thereby allowing a simple one-handed procedure.

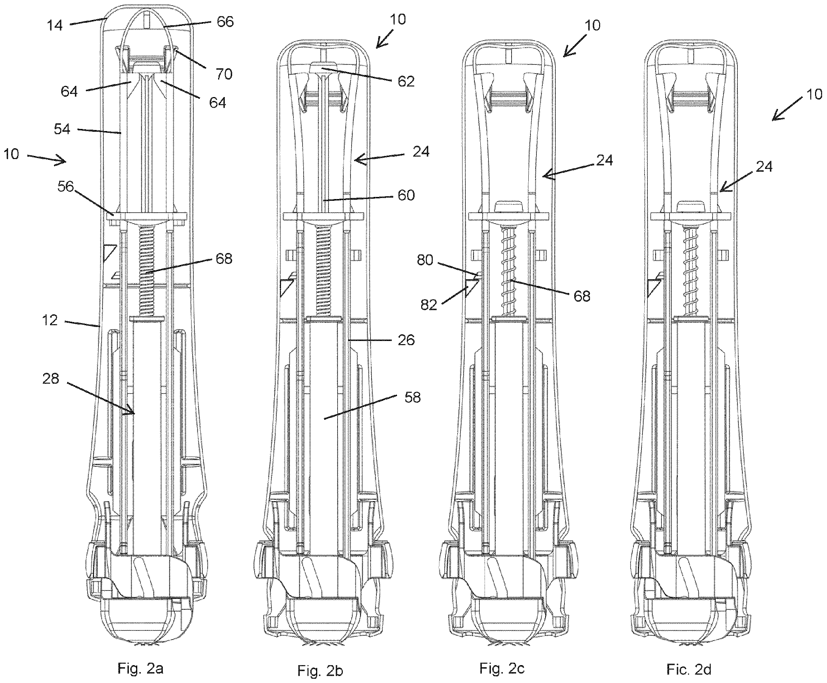

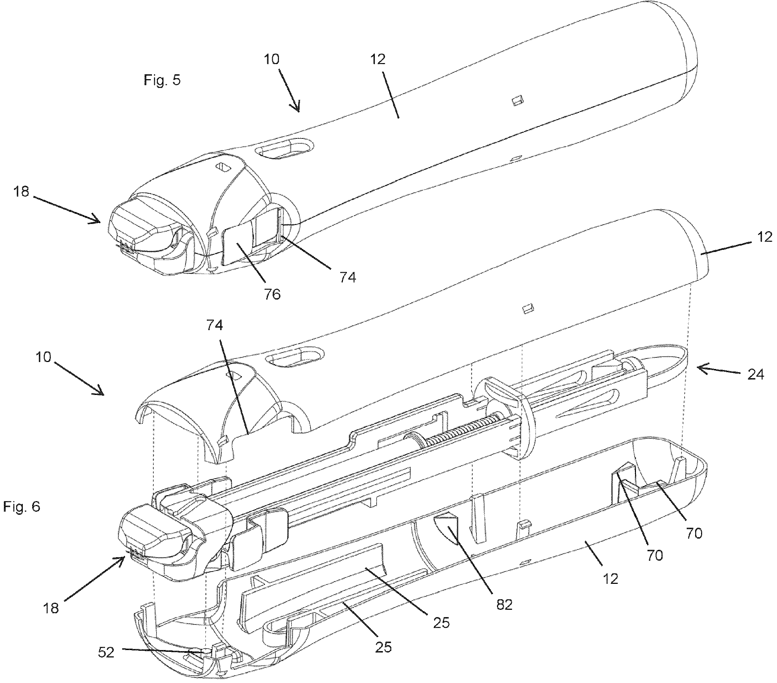

Referring to Figures 2 and 6 the interior of the injection system 10 is shown, the injection system 10 comprising an actuator assembly 24 captured within the housing 12 and which is adapted to undergo longitudinal displacement relative to the housing 12 in order to effect various operational automated functions as described hereinafter. The housing 12 is preferably manufactured as a clam shell construction comprising a pair of halves which are snap fitted or otherwise secured together such as to capture the actuator assembly 24 therebetween, as shown for example in Figure 6. A pair of parallel guiderails 25 may be formed integrally on the interior of the housing 12 in order to guide the actuator assembly 24 during the relative longitudinal displacements.

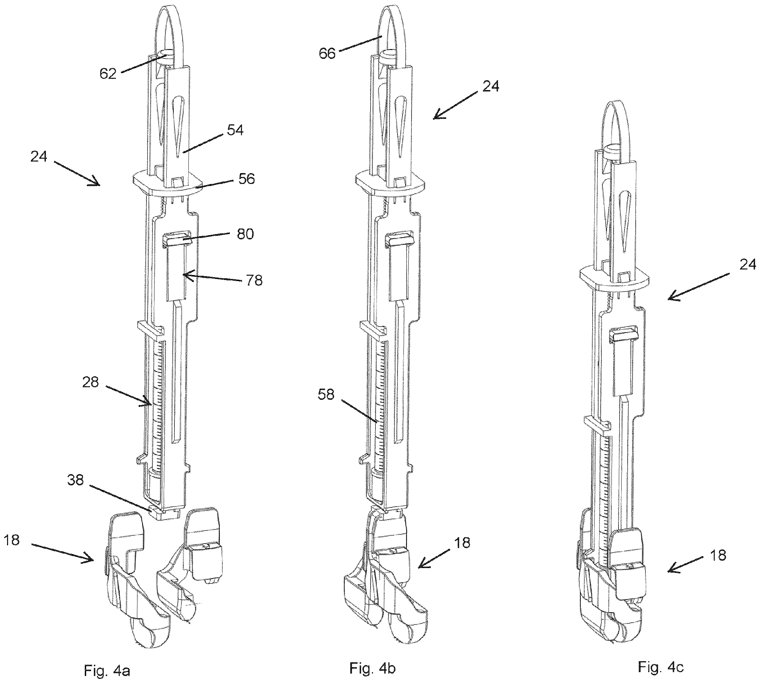

The actuator assembly 24 comprises an elongate frame 26 shown in isolation in Figures 4a to 4c within which is mounted a syringe 28 containing the liquid medicament for injection. The frame 26 is preferably formed from a polymer but any other suitable material may be employed. The injection head 18 is mounted at a lower end of the frame 26 to project from the lower end 16 of the housing 12 and is in fluid communication with the syringe 28. As a result, and referring to both Figures 1 and 2, the housing 12 can therefore be displaced longitudinally relative to the actuator assembly 24 and the injection head 18, from an undeployed state or condition as shown in Figures 1a and 2a, in which state the microneedles 22 are placed in contact with the skin, to a deployed state or condition as shown in Figures 1b and 2b in which state the microneedles 22 are inserted into the skin, through relative longitudinal displacement between the housing 12 and the actuator assembly 24. This is achieved, as described in detail hereinafter, by translating the relative longitudinal displacement between the housing 12 and actuator assembly 24 into relative displacement between the pair of jaws 20 in a direction transverse to the direction of longitudinal displacement. For reference, the injection system 10 is preferably held substantially perpendicular to the skin in use, with a longitudinal axis of the housing 12 defining the longitudinal direction of displacement, and the jaws 20 will therefore undergo displacement transverse to this direction and thus in a direction generally parallel to the skin surface. This transverse or lateral displacement acts to insert the angled microneedles 22 into the skin, with the opposed sets of microneedles 22 overlapping and acting to grip the intervening skin and thus retain or anchor the microneedles 22 in place in order to ensure stability during use and therefore accurate and consistent injection of the liquid medicament. This anchoring action is described in detail in earlier International application WO2018/069543, and no further explanation is therefore necessary in relation to this particular aspect of the invention.

Once the microneedles 22 have been either partially or fully anchored in the skin or other tissue, a final longitudinal displacement of the housing 12 towards the skin and relative to the actuator assembly 24 effects operation of the syringe 28 to inject the liquid medicament through the microneedles 22 and into the surrounding tissue, as will be described in detail hereinafter. This stage is illustrated in Figure 2c and following which the injection system 10 can be manually operated to release the microneedles 22 from the skin to enable the injection system 10 to be disengaged and withdrawn, as shown in Figures 1c and 2d. This action is again described in detail hereinafter.

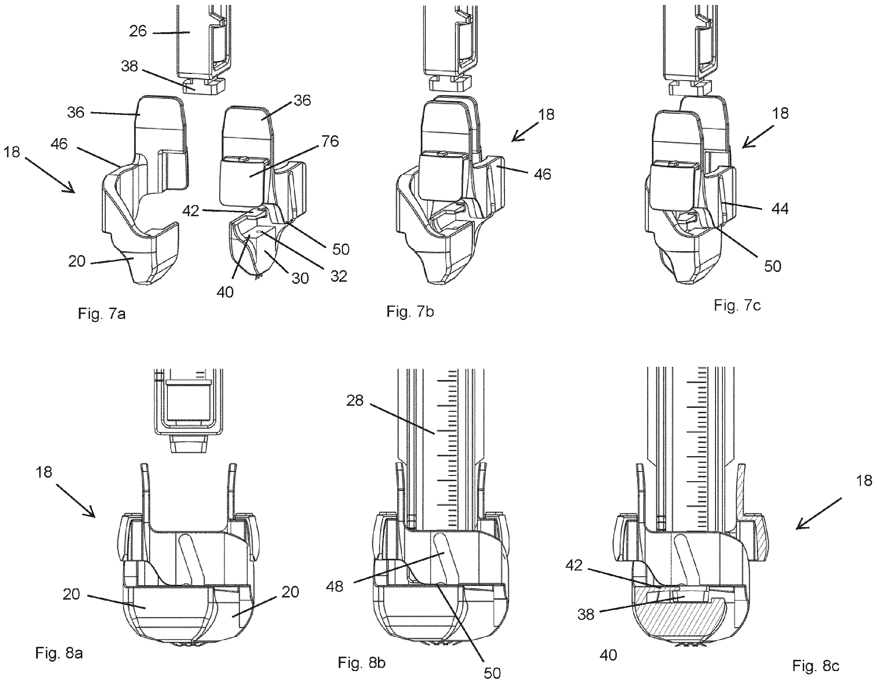

The primary function of the automated injection system 10 is thus to allow a single and simple action, namely using the housing 12 as a handle to press the injection head 18 against the skin to automatically effect a number of operations which together insert the microneedles 22 into the skin and then inject the fluid from the syringe 28 through the embedded microneedles 22. To achieve the first phase of the injection operation the pair of jaws 20 are arranged to undergo transverse or lateral displacement relative to one another in response to the longitudinal displacement of the housing 12 relative to the actuator assembly 24 under the downward pressure applied by the use via the housing 12. The pair of jaws 20 are interlocked with one another, Figure 7a showing the jaws 20 fully separated from one another prior to assembly with the frame 26, Figure 7b showing the jaws 20 being brought together for assembly, while Figure 7c shows the jaws 20 interlocked and ready to receive and be secured to the frame 26 and as are arranged within the housing 12 during operation. Figure 8a shows a front elevation of the arrangement seen in Figure 7c, Figure 8b a front elevation with the frame 26 having been advanced downwardly between the jaws 20, with Figure 8c showing the jaws 20 having being displaced relative to one another such as to be coupled to the frame 26 as hereinafter described.

When interlocked the jaws 20 are capable of undergoing limited translational displacement relative to one another in order to slide the microneedles 22 on each jaw 20 towards one another in order to engage and embed the microneedles 22 into the skin. Each jaw 20 comprises an inner wall 30 which are in face to face contact with one another and thus preferably have a smooth surface finish in order allow the jaws 20 to slide against one another with limited friction. The jaws 20 further comprise an upstanding tab 36 between which is defined an enclosure or space into which a lower end of the frame 26 and a fluid manifold 38 are located, the fluid manifold 38 being captured between an upper face 40 and clamping rail 42 of the pair of jaws 20 to secure the injection head 18 to the frame 26 of the actuator assembly 24. As can be appreciated from Fig 5 the upstanding tab 36 provides a visual shield to hide the internal elements of the device from the user.Tapered contact surfaces on the fluid manifold 38 and clamping rails 42 ensure a fluid tight seal between the fluid manifold 38 and the upper face 40 of the jaws 20. One or more fluid conduits 32 extend from each upper face 40, through the jaw 20, to the respective microneedles 22. In this way a fluid path is established from the syringe 28, through the fluid manifold 38 and jaws 20 to the microneedles 22. An example of this interface between the pair of jaws 20, fluid manifold 38 and syringe 26 is described and shown in detail in the Applicant's International application PCT/EP2022/062653 and no further explanation of the configuration and operation of this aspect of the invention is therefore necessary.

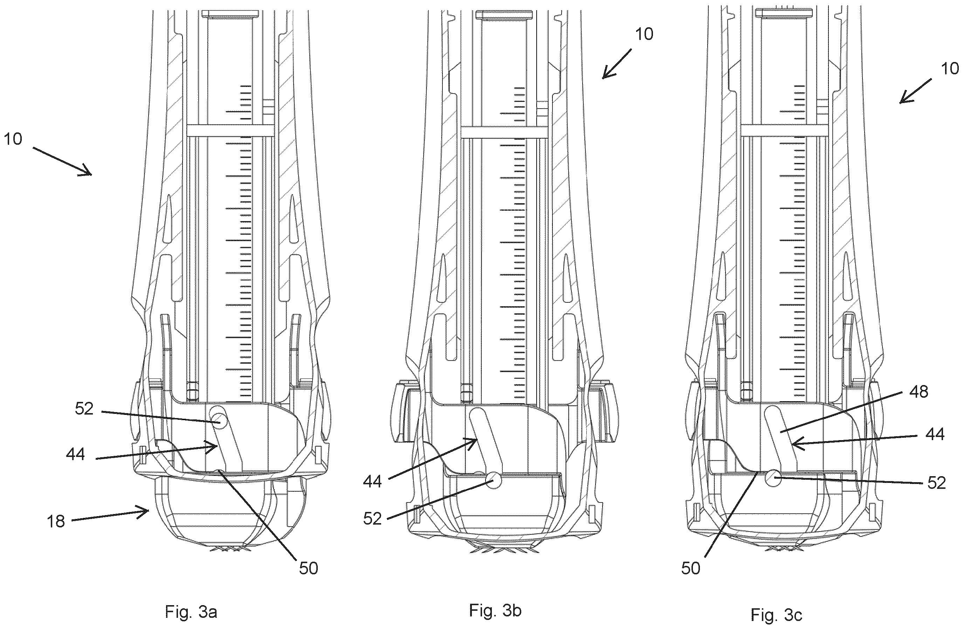

Each jaw 20 further comprises a cam 44 in the form of a channel formed in an outer face 46 of the jaw 20 which is offset from the inner wall 30 and arranged, with the pair of jaws 20 interlocked as shown in figures 7c, to be position above and in line with the lower portion of the opposed jaw 20. The cam 44 is profiled to define a first phase 48 which extends simultaneously down and transversely outward, and a second phase 50 which extends from an open lower end of the channel solely transversely along an underside of the outer face 46 of the jaw 20. The housing 12 is provided with a corresponding pair of followers 52 (see Figures 3 and 6) in the form of a pair of opposed pins 52 each projecting from an inner face of the housing 12 on opposite sides thereof such as to be captured within the respective cam 44. The followers 52 are positioned on the housing 12 such that with the injection system 10 in the undeployed state the follower 52 is seated in the upper end of channel defining the cam 44, for example as shown in Figure 3a, which corresponds to the condition shown in Figure 1a and Figure 2a. It will therefore be understood that relative longitudinal displacement between the housing 12 and the injection head 18, in pressing the housing 12 towards the skin, drives the followers 52 down the cams 44 from the position shown in Figures 2a and 3a to the position shown in Figures 2b and 3b. The orientation of the first phase 48 of the cam 44 will therefore result in the respective jaw 20 being displaced transversely towards the other jaw 20, thereby displacing the microneedles 22 across the skin into which the microneedles 22 are being pressed by virtue of the pressure being applied to the housing 12 by the user. This action effects insertion of the angled microneedles 22 into the skin, and the overlapping arrays of microneedles 22 anchor the injection head 18 to the skin to allow the uninterrupted injection of the liquid medicament from the syringe 28.

The second phase 50 of the cams 44 allow the jaws 20 to be displaced in reverse or away from one another in order to withdraw the microneedles 22 from the skin following injection of the fluid, details of which are provided hereinafter. The second phase 50 of action of the cam 44 and follower 52 occurs once the follower 52 is displaced out of the lower open end of the channel defining the cam 44, whereby the follower 52 can then be displaced transversely relative to the jaw 20, moving along the underside or lower edge of the outer face 46. The second phase 50 may comprise a shallow detent in the lower edge of the outer face 46 to define a stop or limiter to the transverse movement of the jaws 20. It should be understood that the location of the cam 44 and followers 52 could be reversed, with the cam being formed in the wall of the housing 12 and the follower being provided on the exterior face of the jaw 20. It will also be appreciated that the second phase 50 of the cam 44 could be formed as a transverse extension of the channel in the outer face 46 as opposed to being defined beneath the outer face 46.

Turning to the operation of the syringe 28, the frame 24 comprises a pair of elongate side members 54 which together with a cross member 56 and the lower end of the frame 24 define an enclosure in which a barrel 58 of the syringe 28 is located and retained. A dispenser or plunger 60 of the syringe 28 extends through the cross member 56 and terminates in an abutment or head 62 which, with the syringe full and primed for use, is located towards the upper end 14 of the housing 12. The elongate side members 54 extend longitudinally beyond the cross member 56 and although free ends 64 of the side members 54 are connected by a loop of flexible material forming a leaf spring 66, the free ends 64 of the side members 54 effectively define cantilevers which are therefore resiliently deformable in a transverse or lateral direction, away from one another, as described hereinafter. The leaf spring 66 acts against the closed upper end 14 of the housing 12 such as to hold the actuator assembly 24 and housing 12 in the undeployed relative positions. The leaf spring 66 has sufficient bias to hold the housing 12 in said longitudinal position in the absence of external pressure, but is sufficiently weak to be comfortably overcome by a user gripping the housing 12 and pressing the injection head 18 against the skin to effect relative longitudinal displacement of the housing 12 and actuator assembly 24 against the bias of the leaf spring 66. The leaf spring 66 may be formed from any suitable material, and may for example be integrally moulded with the side members 54 from a polymer or the like.

The actuator assembly 24 further comprises a compression spring 68 seated around the plunger 60 and captured and compressed between a piston (not shown) of the syringe 28 and the cross member 56, thereby applying a biasing force to the piston urging the plunger 60 into the barrel 58 such as to force the liquid medicament from the barrel 58. However the free ends 64 of the side members 54 are engaged beneath the head 62 and thus act as latch members preventing the compressed spring 68 from depressing the plunger 60. The actuator assembly 24 comprises a guide member in the form of a pair of ramps 70 projecting from the inner wall of the housing 12 adjacent the upper end 14 and in a position to engage and displace the free ends 64 of the side members 54 transversely and thus out of engagement with the head 62, in response to the longitudinal displacement of the housing 12 relative to the actuator assembly 24. In particular the thin end of each ramp 70 defines a proximal end of the ramp 70 relative to the direction of displacement of the housing 12, which thin end thus initially contacts the end of the side member 54 adjacent the free end 64 with further longitudinal displacement of the housing 12 driving the ramp 70 relative to the side member 54 and effecting the transverse displacement thereof. The side member 54 is resiliently deformable in order to allow this deflection of the free end 64. The side members 54 may for example be formed from any suitable polymer or the like. The ramps 70 are positioned and dimensioned such as to displace the free ends 64 clear of the head 62 only once the pair of jaws 20 have undergone their full relative displacement such that the microneedles 22 are fully embedded in the skin and stabilised. It will be appreciated that the ramps 70 are merely one exemplary form of mechanical arrangement operable to translate the longitudinal displacement of the housing 12 into transverse displacement of the side members 54 or otherwise latching the head 62, and any other functional alternative may be employed.

Once the free ends 64 have been deformed transversely or laterally clear of the head 62 the bias of the spring 68 will be free to drive the piston (not shown) down the barrel 58 of the syringe 28 to force the liquid medicament through the microneedles 22 and into the tissue. Figure 2c shows the injection system 10 at this stage, the free ends 64 deformed outwardly and the plunger 60 having been depressed, such that the spring 68 is uncompressed. The housing 12 is preferably provided with a window 72 adjacent the barrel 58 of the syringe 28 to allow a user to visually confirm that the liquid medicament has been dispensed and thus injected into the skin or other tissue.

At this point the injection system 10 has completed the injection of medicament and should be removed, but the microneedles 22 are still embedded in the skin and must therefore be retracted before the injection system 10 can be disengaged. In order to retract the microneedles 22 it is necessary to reverse the relative transverse displacement of the jaws 20. In order to facilitate this action the housing 12 is provided with a pair of lateral openings 74 proximal the lower end 14, each of the openings 74 being positioned to receive release member in the form of a button 76 projecting from the upstanding tab 36 of each jaw 20. The openings 74 are elongate in the longitudinal direction to accommodate the buttons 76 during the relative longitudinal displacement between the housing 12 and the injection head 18. In addition, as the jaws 20 are displaced laterally together the buttons 76 will be displaced laterally outwardly to project further from the openings 76. The housing 12 may taper inwardly in the region of the openings 76 such as to define a narrow waist at the upper end of the openings 76 in order to further expose the buttons 76 when the housing 12 has been fully depressed against the skin and the injection head 18 has been substantially displaced into the housing 12 through the open lower end 16.

It will therefore be appreciated that once the medicament has been injected the jaws 20 can be displaced back to the undeployed state by the user squeezing the pair of buttons 76 inwardly of the housing 12 which will force the jaws 20 back to the undeployed position. This action will also withdraw the microneedles 12 from the skin allowing the injection system 10 to be disengaged. Figures 1c, 2d, and 3c show the injection system 10 with the buttons 76 depressed back into the housing 12 and the jaws 20 back in the undeployed state. The second phase 50 of each of the cams 44 facilitates this lateral displacement of the jaws 20 without requiring any relative longitudinal displacement between the housing 12 and the injection head 18. It is preferable that the injection system 10 is a single use product that can not be inadvertently or otherwise reused and it is therefore desirable that the housing 12 can not be displaced back into the undeployed state. The actuator assembly 24 therefore comprises a locking element 78 operable to prevent reversal of the relative longitudinal displacement between the housing 12 and the actuator assembly 24. The locking element 78 comprises a resiliently deformable detent 80 formed integrally with one of the side members 54 and projecting laterally therefrom, and a corresponding tab 82 provided on the inner wall of the housing 12. The tab 82 is positioned to contact, deflect and move past the detent 80 during the relative longitudinal displacement between the housing 12 and actuator assembly 24. However the detent 80 and tab 82 are profiled to prevent movement past one another in the reverse direction, in the embodiment illustrated by defining a ratchet type arrangement. In this way the housing 12 will be prevented from returning to the undeployed state once the syringe 28 has been actuated and the medicament dispensed. This is primarily for safety reasons.

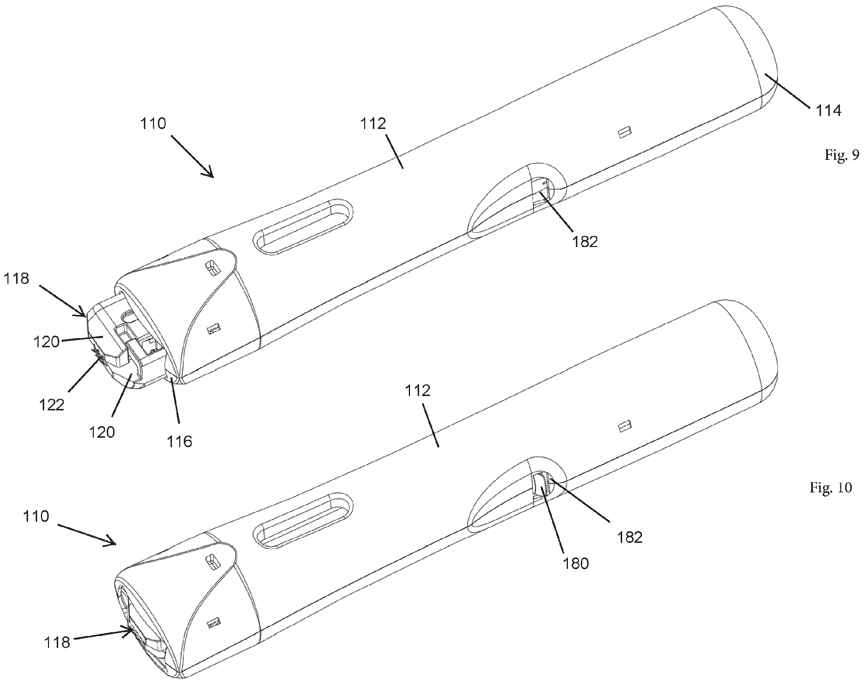

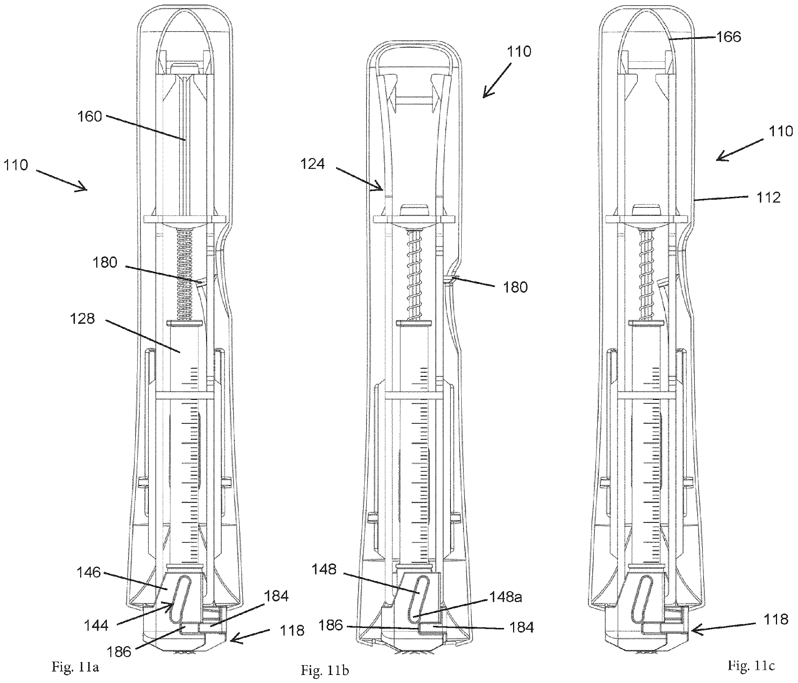

Referring now to Figures 9 to 11 there is illustrated an automated injection system according to an alternative embodiment of the present invention, generally indicated as 110. In this alternative embodiment like components have been accorded like reference numerals and unless otherwise stated perform a like function. The injection system 110 comprises a hollow housing 112 defining a handle, an injection head 118 located at a lower end 116 of the housing 112, and an actuator assembly 124 located within the housing 112. A syringe 128 of a liquid medicament is fixed within the actuator assembly 124 in the same manner as hereinbefore described, and is in fluid communication with one or more microneedles 122 projecting from a pair of jaws 120 of the injection head 118.

In order to achieve the transverse relative displacement of the jaws 120 in response to relative longitudinal displacement of the housing 112 relative to the actuator assembly 124 each jaw 120 comprises a cam 144 formed in an upper wall 146 which in this embodiment is located on the same side of the jaw 120 on which the respective microneedles 122 are provided. The cam 144 includes only a first phase 148 which terminates in a well 148a which is oriented to hold the jaws 120 in a fixed relative position to allow the liquid to be injected through the microneedles 122. Unlike the first embodiment described above the jaws 120 do not include any externally accessible release member to allow reversal of the transverse relative displacement of the jaws 120 such as to extract the microneedles 122 from the skin. Instead the actuator assembly 124 comprises a locking element in the form of a resiliently deformable detent 180 formed integrally with the actuator assembly 124 and projecting laterally therefrom, and a corresponding socket 182 provided in the wall of the housing 112. The tab 180 is positioned to contact a concave section of the housing 112 about the socket 182 and to be deflected inwardly thereby, before reaching and deflecting outwardly into the socket 182 at the end of the relative longitudinal displacement between the housing 112 and actuator assembly 124. Once captured within the socket 182 the detent 180 prevents reversal of the relative movement between the housing 112 and the actuator assembly 124. In this way the housing 112 will be prevented from returning to the undeployed state once the syringe 128 has been actuated and the medicament dispensed. The detent 180 is however externally accessible as clearly seen in Figure 10, allowing the user to depress the detent 180 clear of the socket 182. At this point the housing 112 is free to be displaced longitudinally of the actuator assembly 124, and if the user therefore releases downward pressure against the skin, a leaf spring 166 compressed between a closed upper end 114 of the housing 112 and the actuator assembly 124 will act to reverse the longitudinal displacement between the housing 112 and actuator assembly 124, assisting in pushing the housing 112 upwardly away from the skin. This displacement will therefore reverse the transverse displacement of the pair of jaws 120 to draw the microneedles 122 out of the skin and allow the injection system 110 to be disengaged from the skin. The jaws 120 are each provided with a transversely extending guiderail 184 and a corresponding channel 186 such that when interlocked the guiderail 184 of one jaw 120 is captured within the channel 186 of the other jaw 120. The guiderail 184 and channel 186 are dimensioned to facilitate the relative transverse displacement of the jaws 120 while providing interconnectivity and stability to the jaws 120 during operation.

In a modification to the injection system 110 a plunger 160 of the syringe 128 may be provided with a laterally directed projection (not shown) adapted to engage an inwardly directed projection (not shown) on the deformable detent 180 at or near the terminus of the downward stroke of the plunger 128. This interaction causes the resiliently deformable detent 180 to be brought out of register with the socket 182 in an automated fashion. At this point the housing 112 is free to be displaced longitudinally along the actuator assembly 124, and if the user therefore releases downward pressure against the skin, a leaf spring 166 compressed between the closed upper end 114 of the housing 112 and the actuator assembly 124 will act to reverse the longitudinal displacement between the housing 112 and actuator assembly 124, assisting in pushing the housing 112 upwardly away from the skin.

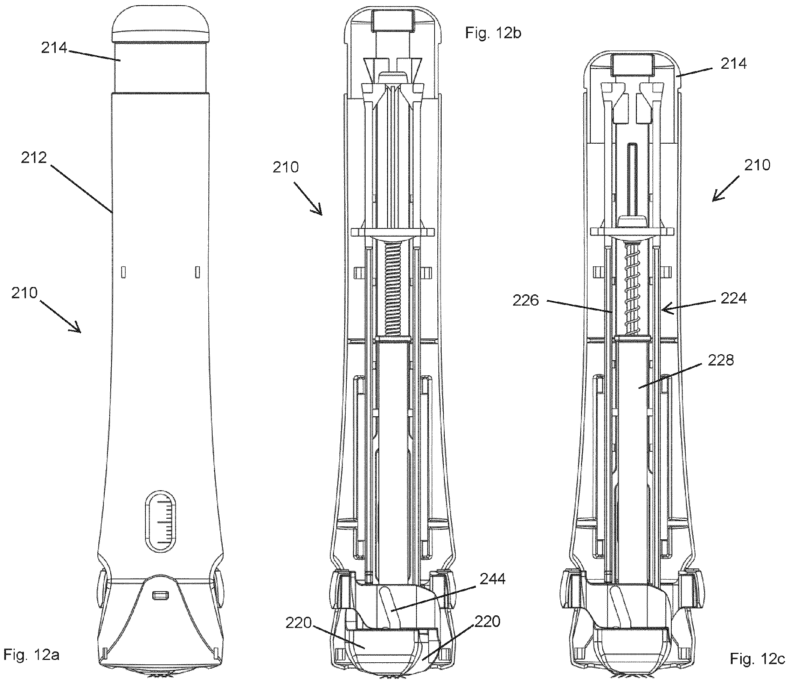

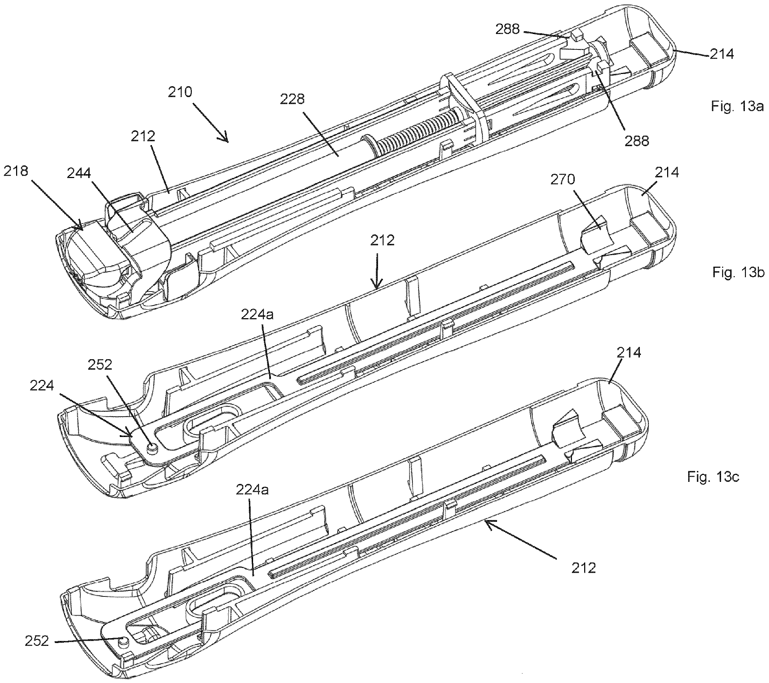

Referring now to Figures 12 and 13 there is illustrated an automated injection system according to a still further alternative embodiment of the present invention, generally indicated as 210. In this alternative embodiment like components have been accorded like reference numerals and unless otherwise stated perform a like function. The injection system 210 comprises a hollow housing 212 defining a handle, an injection head 218 located at a lower end 216 of the housing 212, and an actuator assembly 224 located within the housing 212. The injection head 218 operates as hereinbefore described, including a pair of jaws 220 displaceable transversely relative to one another in response to relative movement between the actuator assembly 224 and the housing 212. The actuator assembly 224 comprises a fixed frame 226 carrying a syringe 228 as hereinbefore described, with a liquid medicament to be dispensed from the jaws 220 in essentially the same manner as described in relation to the previous embodiments. However unlike the previous embodiments the frame 226 is fixed relative to the housing 212, the actuator assembly 224 further comprising a pair of actuator plates 224a each located or captured between the inner wall of the housing 212 and the frame 226 and displaceable longitudinally relative to the housing 212. This relative longitudinal displacement is most clearly seen in Figures 13b and 13c in which the visible actuator plate 224a is respectively located in the undeployed and deployed state. The actuator plates 224a are secured to an upper end 214 of the housing 212, which upper end 214 is telescopically displaceable into the housing 212 in order to effect the longitudinal displacement between the actuator assembly 224 and the dispensing head such as to effect the relative transverse displacement of the jaws 220.

The jaws 220 are therefore each provided with a cam 244 formed therein, a corresponding follower 252 projecting from a corresponding portion of the respective actuator plate 224a. It will therefore be appreciated that by depressing the upper end 214 into the housing 212 the actuator plates 224a will undergo longitudinal displacement relative to the housing 212 and as a result the followers 252 will be driven down the cams 44 to effect the relative transverse displacement of the jaws 220. The actuator assembly 224 comprises a guide member in the form of a pair of ramps 270 projecting from the inner wall of the upper end 214 of the housing 212 and in a position to engage and displace the free ends of the frame 226 in response to the longitudinal displacement of the actuator assembly 224 relative to the housing 212 and as hereinbefore described in relation to the first disclosed embodiment. This will release the spring biased syringed to inject the liquid medicament as hereinbefore described. The free ends of the frame 226 are preferably provided with a locking element in the form of notches or recesses 288 which are located and dimensioned to receive the thick or upper end of the respective ramp 270 once the upper end 214 of the housing 112 has been fully depressed, thereby acting in the manner of a ratchet to prevent the reverse displacement of the upper end 214 and the actuator plates 224a. Following injection the jaws 220 may be manually reversed relative to one another to allow the injection system 210 to be disengaged from the skin.

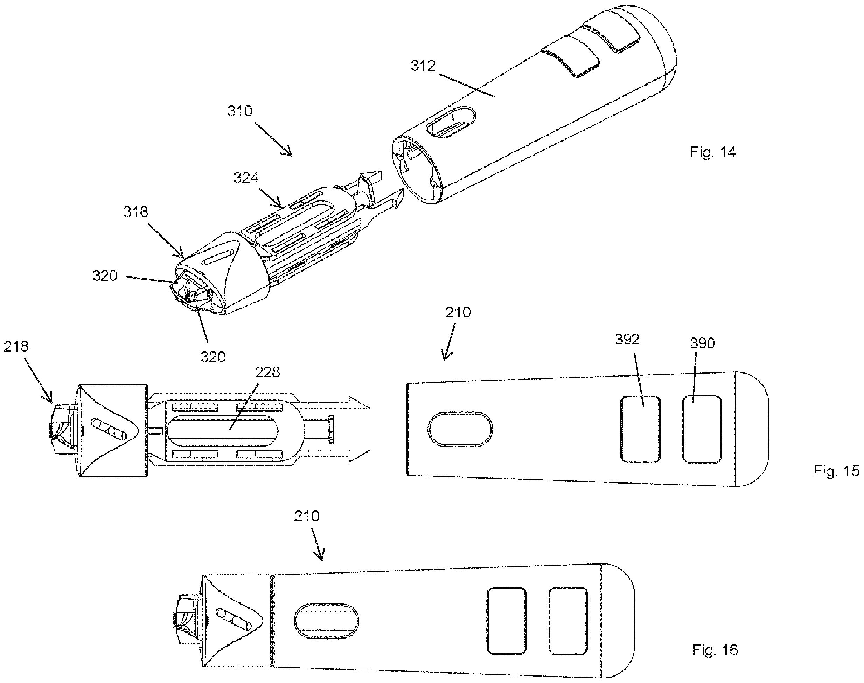

Referring now to Figures 14 to 16 there is illustrated an automated injection system according to an even further alternative embodiment of the invention, generally indicated as 310. In this alternative embodiment like components have been accorded like reference numerals and unless otherwise stated perform a like function. Unlike the previous two embodiments the injection system 310 is intended to be at least partially reusable, and comprises a hollow housing 312 into which is removably locatable an actuator assembly 324 carrying an injection head 318 which operates essentially as hereinbefore described. A syringe 328 of a liquid medicament is fixed within the actuator assembly 324 and is in fluid communication with one or more microneedles 322 projecting from a pair of jaws 320 of the injection head 318. The housing 312 is however provided with one or more motors (not shown) and associated power supply (not shown) such as a battery or the like operable to effect the transverse displacement of the jaws 320 to insert the microneedles 322, and to depress a plunger of the syringe 328. As a result the jaws 320 remain fixed longitudinally with respect to the housing 312, which thus only undergoes relative longitudinal displacement relative to the actuator assembly 324, in particular the syringe 328.

The housing 312 includes a first control or button 390 which may be depressed to activate a motor (not shown) to effect the transverse displacement of the jaws 320 in order to insert the microneedles 322. The injection system 310 may then automatically depress the plunger of the syringe 328, again using a suitable motor (not shown), to inject the medicament. Once the medicament has been injected the injection system 310 may be operable, for example through one or more micro switches or sensors (not shown), to automatically reverse the transverse displacement of the jaws 320 in order to retract the microneedles 322 and allow the injection system 310 to be disengaged from the injection site. The injection system 310 preferably comprises a second button 392 which when depressed allows the actuator assembly 324 and injection head 318 to be released from the housing 312 to be replaced with an actuator 24 containing a syringe 328 of fresh medicament. The housing 312 may comprise embedded circuitry (not shown) and suitable components to facilitate wireless communication with either a phone-based app, cloud-based server or both in order to automatically log injection performance and frequency to enable assessment of patient compliance and/or injection performance issues.

The injection system 10; 110; 210; 310 of the present invention therefore provides a means of effecting intradermal injection which is simple for the user, reliable, repeatable and essentially painless as a result of the use of microneedles.

## Claims

1. An automated injection system comprising an elongate housing defining a handle; an actuator assembly disposed within the housing and displaceable longitudinally relative to the housing; an injection head mounted to the actuator assembly and projecting from a lower open end of the housing, the injection head comprising a pair of jaws displaceable relative to one another in a direction transverse to a longitudinal axis of the housing and in response to the relative longitudinal displacement between the housing and the actuator assembly, each jaw having one or more hollow microneedles projecting therefrom; a reservoir of liquid contained within the housing in fluid communication with one or more of the hollow microneedles; and a dispenser operable to pressurise the fluid in the reservoir in response to the relative longitudinal displacement between the housing and the actuator assembly in order to dispense the liquid from the one or more hollow microneedles.

2. The automated injection system of claim 1 in which the reservoir is defined by a syringe comprising a cylinder closed at one end by a piston displaceable into the cylinder by the dispenser in order to pressurise the fluid.

3. The automated injection system of claim 2 in which the dispenser comprises a spring biased plunger in operative association with the piston.

4. The automated injection system of claim 3 in which the plunger comprises an abutment and the actuator assembly comprises a latch member against which the abutment is releasably retained.

5. The automated injection system of claim 4 in which the actuator assembly is operable to effect displacement of the latch member out of engagement with the abutment prior to or following the completion of relative transverse displacement of the pair of jaws.

6. The automated injection system of claim 5 in which the actuator assembly comprises a biasing element arranged to urge the latch member out of engagement with the abutment in sequence with and/or following the relative longitudinal displacement between the housing and the actuator assembly.

7. The automated injection system of claim 6 in which the biasing element comprises a guide member fixed to the housing and displaceable against the latch member in response to the relative longitudinal displacement between the housing and the actuator assembly such as to urge the latch member out of engagement with the abutment.

8. The automated injection system of any preceding claim in which one of the jaws and the housing comprise a cam and the other of the jaws and the housing comprise a corresponding follower, the cam having a profile arranged to effect the relative transverse displacement of the pair of jaws in response to the relative longitudinal displacement between the housing and the actuator assembly.

9. The automated injection system of claim 8 in which each jaw comprises a cam and the housing comprising a corresponding pair of followers.

10. The automated injection system of any preceding claim in which the injection head comprises at least one release member manually operable to reverse the relative transverse displacement between the pair of jaws.

11. The automated injection system of claim 10 in which the housing comprises an aperture in a sidewall through which the release member is accessible following the relative longitudinal displacement between the housing and the actuator assembly.

12. The automated injection system of any preceding claim comprising a locking element operable to prevent reversal of the relative longitudinal displacement between the housing and the actuator assembly.

13. The automated injection system of any preceding claim in which the actuator assembly comprises an elongate frame about which the housing is supported and within which the reservoir is contained, the injection head being mounted at an end of the frame.

14. The automated injection system of any preceding claim in which the hollow microneedles on each jaw are inclined towards the hollow microneedles on the other jaw.

15. The automated injection system of any preceding claim comprising a cap releasably securable about the lower end of the housing to enclose the injection head.
